Europäisches Patentamt

European Patent Office

Office européen des brevets

(11) Veröffentlichungsnummer : **0 031 108**
**B1**

## EUROPÄISCHE PATENTSCHRIFT

(45) Veröffentlichungstag der Patentschrift :
24.08.83

(21) Anmeldenummer : 80107879.1

(22) Anmeldetag : 13.12.80

(51) Int. Cl.³ : **C 07 C 59/185**, C 07 C 59/205,
C 07 C 59/84, C 07 C 51/373

(54) Verfahren zur Herstellung von 5-Oxoalkansäuren.

(30) Priorität : 22.12.79 DE 2952044

(43) Veröffentlichungstag der Anmeldung :
01.07.81 Patentblatt 81/26

(45) Bekanntmachung des Hinweises auf die Patenterteilung : 24.08.83 Patentblatt 83/34

(84) Benannte Vertragsstaaten :
BE CH DE FR GB IT LI NL

(56) Entgegenhaltungen :
EP A 0 008 482

JOURNAL OF THE CHEMICAL SOCIETY, Heft 12,
1978, LONDON (GB) I. CASINOS et al. : « Synthesis of Unsaturated 5-Hydroxy- and 7-Oxoacids
by Addition of Unsaturated Carboxylic Acid Dienolates to Carbonyl Compounds », Seiten 1651-
1655

(73) Patentinhaber : HOECHST AKTIENGESELLSCHAFT
Postfach 80 03 20
D-6230 Frankfurt am Main 80 (DE)

(72) Erfinder : Müller, Werner Heinrich, Dr.
Taunusblick 5
D-6239 Eppstein/Taunus (DE)

## Verfahren zur Herstellung von 5-Oxoalkansäuren

Es ist bekannt, daß man 5-Oxoalkansäuren bzw. deren Ester oder Nitrile durch Addition eines Ketons, das zumindest ein Wasserstoffatom in $\alpha$-Position zur Ketogruppe enthält, an $\alpha,\beta$-ungesättigte Säuren, Ester oder Nitrile herstellen kann, wenn ein primäres Amin als Katalysator verwendet wird (z. B. DE-PS 2 329 923, DE-OS 2 325 160, DE-OS 2 355 859, DE-PS 2 348 536, DE-PS 2 540 972). Bei der Herstellung der 5-Oxoalkansäuren nach dieser Methode erweist sich die erhöhte Bildung von Ketonkondensationsprodukten als besonders nachteilhaft. Im Falle der Herstellung von 5-Oxohexansäure aus Aceton und Acrylsäure können bis zu 50 % des verbrauchten Acetons in Mesityloxid umgewandelt werden. Man kann zwar durch Arbeiten bei erhöhter Mesityloxidkonzentration im Reaktionsgemisch die weitere Bildung von Mesityloxid zurückdrängen, doch bedeutet dies erhöhten Aufwand wegen der notwendigen Abtrennung von Mesityloxid aus dem Reaktionsprodukt und Rückführung. Außerdem besteht bei erhöhter Mesityloxid-Konzentration die Gefahr der Nebenproduktbildung durch Weiterreaktion von Mesityloxid mit Aceton, Acrylsäure oder Amin.

Es bestand daher die Aufgabe, bei der Herstellung von 5-Oxoalkansäuren die Kondensation der verwendeten Ketone zu Nebenprodukten zurückzudrängen und auch die Selektivität bezogen auf verbrauchte $\alpha,\beta$-ungesättigte Säure zu erhöhen.

Es wurde nun ein Verfahren gefunden zur Herstellung einer 5-Oxoalkansäure durch Addition eines Ketons, das zumindest ein Wasserstoffatom in $\alpha$-Stellung zur Ketogruppe enthält, an eine $\alpha,\beta$-ungesättigte Säure bei erhöhter Temperatur, dadurch gekennzeichnet, daß man die Reaktion in Gegenwart eines sekundären Amins als Katalysator ausführt.

Bisher waren für die Herstellung der 5-Oxoalkansäuren bzw. deren Estern oder Nitrilen aus Ketonen und $\alpha,\beta$-ungesättigten Säuren bzw. deren Estern oder Nitrilen speziell primäre Amine oder Verbindungen mit primärer Aminogruppe als Katalysatoren eingesetzt worden.

Es war deshalb sehr überraschend, daß sekundäre Amine bei der 5-Oxoalkansäure-Herstellung sogar etwas aktiver sind als primäre Amine. Gleichzeitig werden weniger Nebenprodukte durch Eigenkondensation der verwendeten Ketone gebildet, und die Selektivität der Oxoalkansäuren bezogen auf verbrauchte $\alpha,\beta$-ungesättigte Säure ist höher.

In Beyer, Lehrbuch der organischen Chemie, S. 284 und W. L. Allinger et al., Organische Chemie, S. 1221 werden sekundäre Amine als Katalysatoren für die Michael-Addition bezeichnet, es ist aber zumindest zweifelhaft, ob diese Autoren die vorliegende Umsetzung von freien $\alpha,\beta$-ungesättigten Carbonsäuren mit bestimmten Ketonen als Michael-Addition auffassen. Außerdem ist selbst bei wirklichen Michael-Additionen der Einsatz sekundärer Amine mit Reaktionszeiten von 20 bis 48 Stunden verbunden, wie aus Bergmann et al., The Michael Reaction, Org. Reactions *10*, 1959, S. 266 unten, hervorgeht.

Nach J. Chem. Soc. *12*, 1979, kann man 7-oxo-Alkansäuren aus $\alpha,\beta$-ungesättigten Säuren und einem Keton herstellen. Als Katalysator wird jedoch kein sekundäres Amin, sondern ein aus diesem hergestelltes Lithiumamid verwendet.

Für die Umsetzung verwendet man Ketone, die in $\alpha$-Stellung zur Ketogruppe mindestens ein Wasserstoffatom besitzen, beispielsweise Aceton, Methylethylketon, Diethylketon, Methylpropylketon, Methylisopropylketon, Hexanon-2, Heptanon-2, Octanon-2, Nonanon-2, Acetylaceton, Acetonylaceton, Cyclopentanon, Cyclohexanon, Acetophenon, Propiophenon und Phenylaceton.

Als geeignete $\alpha,\beta$-ungesättigte Säuren seien beispielsweise genannt :

Acrylsäure, Methacrylsäure, Maleinsäure, Fumarsäure und Itakonsäure.

Als Katalysatoren sind sekundäre aliphatische und cycloaliphatische Amine mit $C_1$-$C_8$-Alkylgruppen besonders geeignet, beispielsweise Dimethylamin, Methyl-ethylamin, Diethylamin, Dipropylamin, Diisopropylamin, Dibutylamin, Diisobutylamin, N-Ethylbutylamin, Di-(2-ethylhexyl)-amin, N-Cyclohexylmethylamin, Piperidin, Pyrrolidin, Morpholin und N-Methylpiperazin. Die Menge des eingesetzten Katalysators beträgt im allgemeinen zwischen 0,03 und 0,3 Mol pro Mol $\alpha,\beta$-ungesättigte Säure.

Das molare Verhältnis von Keton zu ungesättigter Säure kann in weiten Grenzen variieren ; es beträgt im allgemeinen 1 : 1 bis 20 : 1, vorzugsweise 2 : 1 bis 8 : 1.

Die günstigste Reaktionstemperatur hängt von der Art und Menge des verwendeten Ketons, der $\alpha,\beta$-ungesättigten Säure und des verwendeten Katalysators ab. Im allgemeinen wird zwischen 100 und 250 °C, vorzugsweise zwischen 150 °C und der kritischen Temperatur der verwendeten Reaktionspartner gearbeitet.

Der Druck wird im allgemeinen zwischen dem der Reaktionstemperatur entsprechenden Dampfdruck und 300 bar, vorzugsweise zwischen Dampfdruck und 100 bar eingestellt.

Die Druckerhöhung über den Dampfdruck der Reaktionskomponenten hinaus kann bewirkt werden durch den eigenen Flüssigkeitsdruck, der entsteht, wenn man den für die Umsetzung benutzten Reaktor bei Raumtemperatur mindestens so weitgehend mit den Reaktionskomponenten füllt, daß beim Erhitzen auf die Reaktionstemperatur die Gasphase verschwindet. Eine andere geeignete Methode, insbesondere bei höher siedenden Ketonen (mehr als 6 C-Atome) besteht darin, daß man ein Inertgas, wie z. B. $N_2$ oder Argon unter einem Druck in den Reaktor einpreßt, der den Dampfdruck der Reaktionskomponenten überschreitet.

Das Arbeiten bei erhöhtem Druck wirkt sich vor allem in der Steigerung der Raumzeitausbeute (g/lh) aus. Die Umsetzung kann mit oder ohne Lösungs- oder Verdünnungsmittel ausgeführt werden. Zweckmäßigerweise wird ein Polymerisationsinhibitor, wie z. B. Hydrochinon, Hydrochinonmonomethylether oder Phenothiazin zugesetzt.

Das erfindungsgemäße Verfahren kann bei diskontinuierlicher Arbeitsweise beispielsweise wie folgt betrieben werden : Die Reaktionskomponenten werden bei Raumtemperatur miteinander vermischt und in einen Reaktor, wie z. B. einen Autoklaven oder ein Schießrohr eingefüllt und für eine bestimmte Zeit auf die gewünschte Reaktionstemperatur erhitzt.

Danach wird das Reaktionsgemisch schnell abgekühlt, analysiert und destillativ aufgearbeitet.

Bei kontinuierlicher Arbeitsweise kann das erfindungsgemäße Verfahren vorteilhaft wie folgt durchgeführt werden :

Ein Gemisch bestehend z. B. aus Aceton, Acrylsäure, Polymerisationsinhibitor und Diethylamin wird nach guter Durchmischung durch ein auf die gewünschte Reaktionstemperatur erhitztes Reaktionsrohr gepumpt. An dessen Ausgang ist eine automatische Stand- und Druckhaltung angebracht. Durch Inertgasüberlagerung wird der gewünschte Reaktionsdruck eingestellt. Das Reaktionsgemisch wird sofort kontinuierlich destilliert. Die unumgesetzten Ausgangsmaterialien werden sofort zurückgeführt.

Vergleichsbeispiel (mit primärem Amin)

Ein Schießrohr von 30 ml Inhalt wird mit 24 ml der nachfolgenden Ausgangsmischung gefüllt, verschlossen und 60 Minuten in ein 230 °C heißes Ölbad versenkt, daraufhin wird abgekühlt und analysiert.

Es werden hier und in den weiteren Beispielen folgende Abkürzungen benutzt :

Sel X/Y = Mol Endprodukt X pro Mol verbrauchtes Ausgangsmaterial Y (Mol-%)
Ac = Aceton
IPA = Isopropylamin
AS = Acrylsäure
OHS = 5-Oxohexansäure
HQ = Hydrochinon
MO = Mesityloxid

| Ausgangsgemisch (Gew.-%) | Ac | AS | IPA | HQ |
|---|---|---|---|---|
| | 78,9 | 19,6 | 1,43 | 0,05 |

| Produktzusammensetzung (Gew.%) | | | | Sel | | |
|---|---|---|---|---|---|---|
| Ac | AS | OHS | MO | OHS/AS | OHS/Ac | MO/Ac |
| 62,7 | 4,9 | 19,1 | 5,2 | 72 | 53 | 38 |

Beispiele 1-3

Versuchsausführung wie im Vergleichsbeispiel, jedoch werden verschiedenen sekundäre Amine als Katalysatoren benutzt, nämlich :

(siehe die Tabelle Seite 4)

0 031 108

Beispiel 1 : Piperidin — Pip
Beispiel 2 : Morpholin — Morph
Beispiel 3 : Diethylamin — DEA

| Beispiel | Ausgangsgemisch (Gew.%) | | | | Reaktionsprodukt (Gew.%) | | | | Sel | | |
|---|---|---|---|---|---|---|---|---|---|---|---|
| | Ac | AS | Katalysator | HQ | Ac | AS | OHS | MO | OHS/AS | OHS/Ac | MO/Ac |
| 1 | 78,6 | 19,5 | Pip 1,8 | 0,05 | 65,8 | 5,6 | 20,3 | 3,0 | 80 | 68 | 26 |
| 2 | 78,4 | 19,5 | Morph 2,0 | 0,05 | 66,2 | 6,8 | 19,3 | 2,5 | 84 | 71 | 24 |
| 3 | 78,7 | 19,5 | DEA 1,7 | 0,05 | 64,8 | 4,9 | 22,4 | 2,5 | 85 | 72 | 21 |

Beispiele 1-3 zeigen die erhöhte OHS-Selektivität durch die erfindungsgemäße Verwendung sekundärer Amine gegenüber dem Einsatz primärer Amine als Katalysatoren gemäß dem Vergleichsbeispiel.

Beispiel 4

(Herstellung von 3-(2-Oxo-cyclohexyl)-propansäure)

24 ml eines Gemisches, bestehend aus 20 g Cyclohexanon, 7.7 g Acrylsäure und 1.5 g Diethylamin, werden in einem 30 ml Schießrohr 30 Minuten lang auf 230 °C erhitzt, abgekühlt und gaschromatographisch analysiert. Das Reaktionsprodukt enthält neben unumgesetzten Ausgangsmaterialien 40 % 3-(2-Oxocyclohexyl)-propansäure, 11 % 3-(2-Oxo-cyclohexyl)-propansäure-diethylamid (Kp 139 °C bei 1.2 mbar) sowie 4 % Cyclohexenylcyclohexanon.

Beispiel 5

(Herstellung von 4-Phenyl-5-Oxohexansäure)

75 g einer Mischung, bestehend aus 74.4 % Benzylmethylketon, 21.6 % Acrylsäure und 4.1 % Diethylamin, werden 30 Minuten lang auf 230 °C erhitzt, abgekühlt und gaschromatographisch analysiert. Das Reaktionsprodukt enthält neben unumgesetzten Ausgangsmaterialien 20 % 4-Phenyl-5-Oxohexansäure (Kp 141-143 °C bei 2 mbar), sowie 5 % einer unbekannten Verbindung mit längerer Retentionszeit als 4-Phenyl-5-Oxohexansäure.

**Anspruch**

Verfahren zur Herstellung einer 5-Oxoalkansäure durch Addition eines Ketons, das zumindest ein Wasserstoffatom in $\alpha$-Stellung zur Ketogruppe enthält, an eine $\alpha,\beta$-ungesättigte Säure bei erhöhter Temperatur, dadurch gekennzeichnet, daß man die Reaktion in Gegenwart eines sekundären Amins als Katalysator ausführt.

**Claim**

A process for the manufacture of a 5-oxoalkanoic acid by addition of a ketone containing at least one hydrogen atom in the $\alpha$-position with regard to the keto group, to an $\alpha,\beta$-unsaturated acid, at elevated temperature, characterized by carrying out the reaction in the presence of a secondary amine as catalyst.

**Revendication**

Procédé de préparation d'un acide oxo-5 alcanoïque par fixation, sur un acide insaturé en $\alpha,\beta$, d'une cétone contenant au moins un atome d'hydrogène en position $\alpha$ par rapport au radical oxo, à température élevée, procédé caractérisé en ce qu'on effectue la réaction en présence d'une amine secondaire servant de catalyseur.